# EUROPEAN PATENT SPECIFICATION

(11) **EP 3 243 051 B1**
(45) Date of publication and mention of the grant of the patent: **24.03.2021**
(21) Application number: 15876729.3
(22) Date of filing: 15.12.2015
(51) Int. Cl.: G01J 4/00, G02B 7/09, G02B 7/28, G03B 3/00, G03B 13/36, G03B 13/34, H04N 5/232

(54) **METHOD, APPARATUS AND COMPUTER PROGRAM FOR DETERMINING FOCUSING INFORMATION**
VERFAHREN, VORRICHTUNG UND COMPUTERPROGRAMM ZUR BESTIMMUNG VON FOKUSSIERUNGSDATEN
PROCÉDÉ, APPAREIL ET PROGRAMME INFORMATIQUE POUR DÉTERMINER DES INFORMATIONS DE FOCALISATION

(30) Priority: 09.01.2015 GB 201500300
(43) Date of publication of application: 15.11.2017
(73) Proprietor: Nokia Technologies Oy, 02610 Espoo (FI)
(72) Inventor: HARRIS, Nadine, Papworth Everard Cambridge CB23 3GT (GB)
(74) Representative: Swindell & Pearson Limited
(86) International application number: PCT/FI2015/050884
(87) International publication number: WO 2016/110609

(56) References cited:
- GB-A- 2 409 033
- JP-A- H1 089 953
- JP-A- H07 222 719
- JP-A- H07 222 719
- JP-A- 2009 168 995
- JP-A- 2013 057 769
- US-A- 5 973 737
- US-A- 5 973 737
- US-A1- 2004 165 099
- US-A1- 2007 247 624
- US-A1- 2008 160 287
- US-A1- 2010 149 073

## Description

### TECHNOLOGICAL FIELD

Examples of the present disclosure relate to a method, apparatus and computer program for controlling a focusing mechanism. Some examples, though without prejudice to the foregoing, relate to auto-focusing systems and a method, apparatus and computer program for controlling the focus of a focusing element.

### BACKGROUND

Conventional auto-focusing systems are not always optimal. Typically, auto-focusing systems determine auto-focusing requirements via the use of a camera taking an image of an object scene that is to be focussed and performing complex image processing and analysis of the captured image to determine focusing adjustments of the camera's lenses that would be required to bring the object scene into correct focus.

The listing or discussion of any prior-published document or any background in this specification should not necessarily be taken as an acknowledgement that the document or background is part of the state of the art or is common general knowledge. One or more aspects/examples of the present disclosure may or may not address one or more of the background issues.

JPH1089953A discloses a focus detecting apparatus whose focusing accuracy is high and wherein the time required for a focusing operation is short. Probing light from a semiconductor laser is reflected by a polarization beam splitter, it is transmitted through a quarter-wave plate and an image formation lens, and it is condensed by an objective lens so as to irradiate an object to be inspected. Its reflected light is transmitted through the objective lens, the image formation lens, the quarter-wave plate and the polarization beam splitter so as to be divided into two beams of light. One beam of light is incident on a light-receiving element via a diaphragm situated at the front of a light focusing point Q, and the other beam of light is incident on a light-receiving element via a diaphragm situated at the rear of the light focusing point Q. A signal processing system generates a displacement signal of (A-B)/(A+B) on the basis of output signals of the light-receiving elements. A laser-position control part adjusts the position in the optical-axis direction of the semiconductor laser on the basis of a signal of (A+B).

US5973737 discloses an apparatus with an illuminating light source for illuminating a user's eye and an optical system for causing a Purkinje image and an anterior eye part image created by the illumination to be formed on a solid state image pickup element, and is designed such that the influence of extraneous light is mitigated in the output signal of the solid state image pickup element by the use of a polarizing element for selecting the polarization characteristic of the image light received by the solid state image pickup element.

JP1972022719A discloses a line of sight detector that seeks to detect a Purkinje image by securing an S/N without depending on the change of brightness when photography is performed. A light projecting part emits light to an eyeball via a first polarizing part based on the signal of a line of sight timing control part and information with respect to the brightness of a photometric part, and the line of sight timing control part generates plural light projection patterns and light projection timings. A light receiving part converts reflected from the eyeball to an electrical signal by receiving correspondingly to the control signal of the line of sight timing control part via a polarizing characteristic variable part and a second polarizing part, and outputs it to a signal processing part. At this time, polarizing characteristic variable part changes the polarizing plane of luminous flux corresponding to the control signal of the line of sight timing control part. The signal processing part performs differential processing based on the signal of the light receiving part, and samples a line of sight feature signal. Also, the device is constituted in such a way that the degree of polarization of the first polarizing part can be set higher than that of the second polarizing part.

### BRIEF SUMMARY

The present invention is as set out in the independent claims.

According to certain examples of the disclosure there is provided a method comprising causing, at least in part, actions that result in: receiving at least one measurement of the polarization of light reflected from at least one surface; and controlling a focusing mechanism dependent, at least in part, on the at least one polarization measurement.

According to certain examples of the disclosure there is provided an apparatus comprising means configured to cause the apparatus at least to perform the above method.

According to certain examples of the disclosure there is provided an apparatus comprising at least one processor; and at least one memory including computer program code; the at least one memory and the computer program code configured to, with the at least one processor, cause the apparatus at least to perform the above method.

According to certain examples of the disclosure there is provided a computer program that, when performed by at least one processor, causes the above method to be performed.

According to certain examples of the disclosure there is provided a non-transitory computer readable medium encoded with instructions that, when performed by at least one processor, causes the above method to be performed.

According to certain examples of the disclosure there is provided a chipset or module comprising processing circuitry configured to: receive at least one measurement of the polarization of light reflected from at least one surface; and control a focusing mechanism dependent, at least in part, on the at least one polarization measurement.

According to certain examples of the disclosure, there is provided a method comprising determining a focusing state of a user's eye based, at least in part, on one or more measurements of the polarization of light reflected from a part of a user's eye.

According to certain examples of the disclosure, there is provided a method comprising: determining a change in polarization of light reflected from a part of a user's eye; and controlling of a focusing mechanism dependent, at least in part, on the determined change in polarization.

The examples of the present disclosure and the accompanying claims may be suitably combined in any manner apparent to one of ordinary skill in the art, without departing from the scope of the claims.

### BRIEF DESCRIPTION OF THE DRAWINGS

For a better understanding of various examples of the present disclosure that are useful for understanding the detailed description and certain embodiments of the invention, reference will now be made by way of example only to the accompanying drawings in which:
Figure 1 schematically illustrates an example of a method of the present disclosure;
Figure 2 schematically illustrates a further example of a method of the present disclosure;
Figure 3 schematically illustrates an example of an apparatus of the present disclosure for measuring the polarization of light reflected from a part of a user's eye when looking at object A;
Figure 4 schematically illustrates the apparatus of Figure 3 measuring the polarization of light reflected from a part of the user's eye when looking at object B;
Figure 5 schematically illustrates an example of a detector for use with apparatuses of the present disclosure;
Figure 6 schematically illustrates an extinction spectrum of a nanoparticle configured to detect polarized light;
Figure 7 schematically illustrates an example of an apparatus of the present disclosure;
Figures 8A and 8B schematically illustrate further examples of an apparatus and a system of the present disclosure; and
Figure 9 schematically illustrates a flowchart depicting operation of a real time autofocusing detector of the present disclosure.

### DETAILED DESCRIPTION

The present invention is as set out in the independent claims.

The Figures schematically illustrate a method (100) comprising:
receiving (101) at least one measurement of a polarization (P_{A}) of light (307) reflected from at least one surface (305', 305") of a part of a user's eye (304);
determining a change in the polarization (ΔP) of the reflected light with respect to the polarization of the incident light (306); and
   and
controlling a focusing mechanism (303) dependent, at least in part, on the determined change in polarization.

The at least one surface may comprise a lens of a user's eye.

The focusing mechanism may comprise means or a device for adjusting the focus of an optical system.

By way of an overview, in certain examples, a change in the polarization of light reflected from a lens of a user's eye, while the eye is focusing, is determined and a focusing mechanism of an optical system is controlled based on the determined change in polarization. Certain examples of the present disclosure make use of an unexpected relationship between the polarization characteristics of light reflected from the user's lens and the user's focus/point of focus. Various examples use polarization characteristics of reflected light to control a focussing mechanism so as to provide a new and improved autofocussing method and apparatus.

Certain examples of the disclosure make use of the eye's natural ability to focus on objects and also make use of measurement of polarization of light reflected from the user's eye for deriving focusing information for controlling a focusing mechanism to provide an improved autofocusing system.

The lens within an eye is flexible and the shape of the lens varies as a function of the distance of the object to be focused. For example, the lens becomes more rounded to focus on near objects and more elongated to focus on more distant objects. Examples of the present disclosure may determine a change in the polarization of light reflected from a lens of a user's eye to indicate a variation in the geometry of the lens whilst it is focusing on an object. Control of a focussing mechanism is effected which is dependent upon the detected change in polarization, thereby controlling one or more focusing elements of an optical device/optical system that is to be focused.

In certain other particular examples, the method and apparatus may involve the use of a detector for detecting the polarization of reflected light, wherein the detector comprises a plurality of nanoparticles configured to have plasmonic resonances that are excitable in response to the polarization state of incident light. The use of such a detector may enable a sufficiently high sensitivity of detection of changes in a polarization state of incident light such that ambient light (such as ambient infrared light) reflected from a lens of a user's eye may be detected. This may avoid the need for a specific/dedicated illumination source for whose polarization is to be measured, such that a simple, light and non-cumbersome apparatus may be provided that may provide fast/real time auto-focusing control.

An example of a method and apparatus according to the present disclosure will now be described with reference to the Figures. Similar reference numerals are used in the Figures to designate similar features. For clarity, all reference numerals are not necessarily displayed in all Figures.

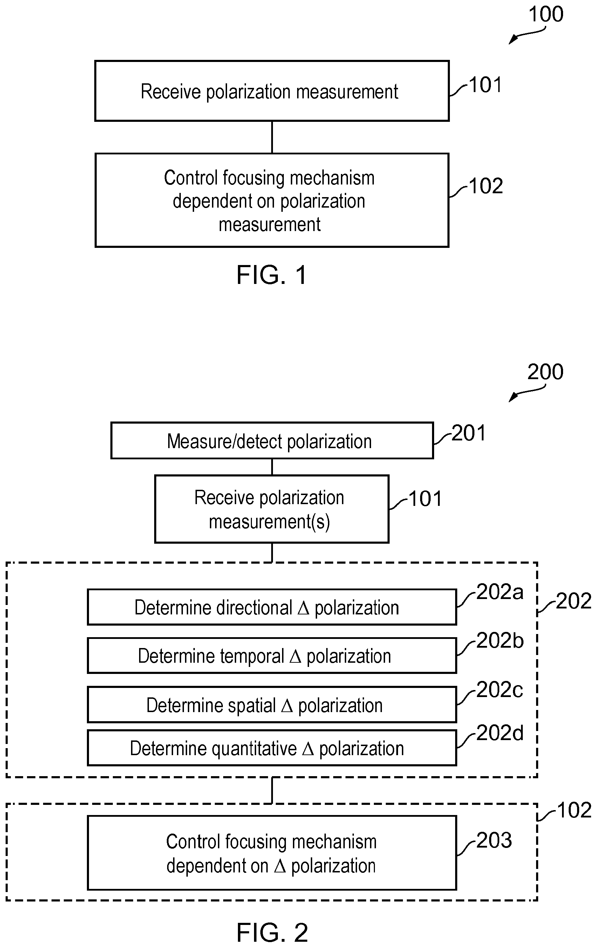

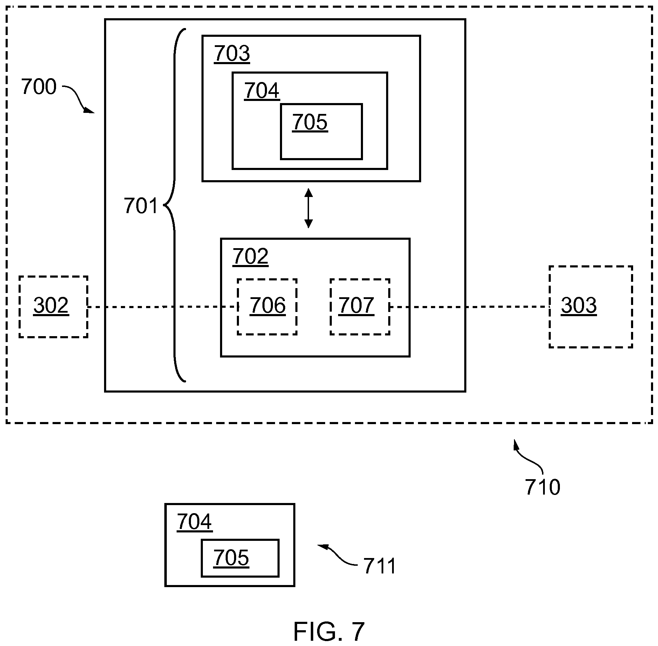

Figure 1 schematically illustrates a method 100 according to an example of the present disclosure. The component blocks of Figure 1 are functional and the functions described may or may not be performed by a single physical entity (such as is described with reference to Figure 7).

In block 101, at least one measurement of the polarization of light reflected from at least one surface is received. In block 102, a focusing mechanism is controlled dependent, at least in part, on the at least one polarization measurement.

The at least one surface may comprise one or more of: a part of a user's eye, and a lens of a user's eye. For example, the at least one surface may comprise a front and/or rear surface of a lens of the user's eye. In certain examples, the surface may comprise a surface of a ciliary muscle.

The polarization measurement may correspond to change in the polarization of reflected light, e.g. a change in the polarization characteristics of the reflected light.

The focusing mechanism may comprise means or a device for adjusting the focus of an optical system. In some examples the focusing mechanism may comprise an actuator configured to move a lens of an optical system. In some examples the focusing mechanism may comprise a mechanism for adjusting the refractive index of a focussing element.

The control of the focusing mechanism may be via generating a control signal for controlling the focusing mechanism, wherein the control signal is generated dependent, at least in part, on the at least one polarization measurement.

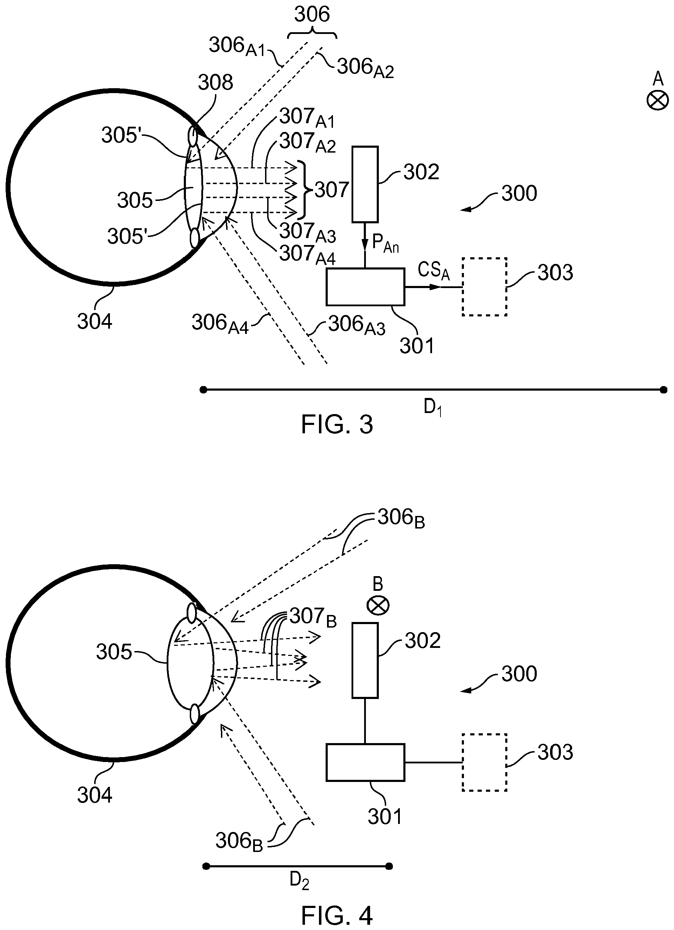

Figure 2 schematically illustrates further method 200 according to another example of the present disclosure. The component blocks of Figure 2 are functional and the functions described may or may not be performed by a single physical entity (such as is described with reference to Figures 3 and 4).

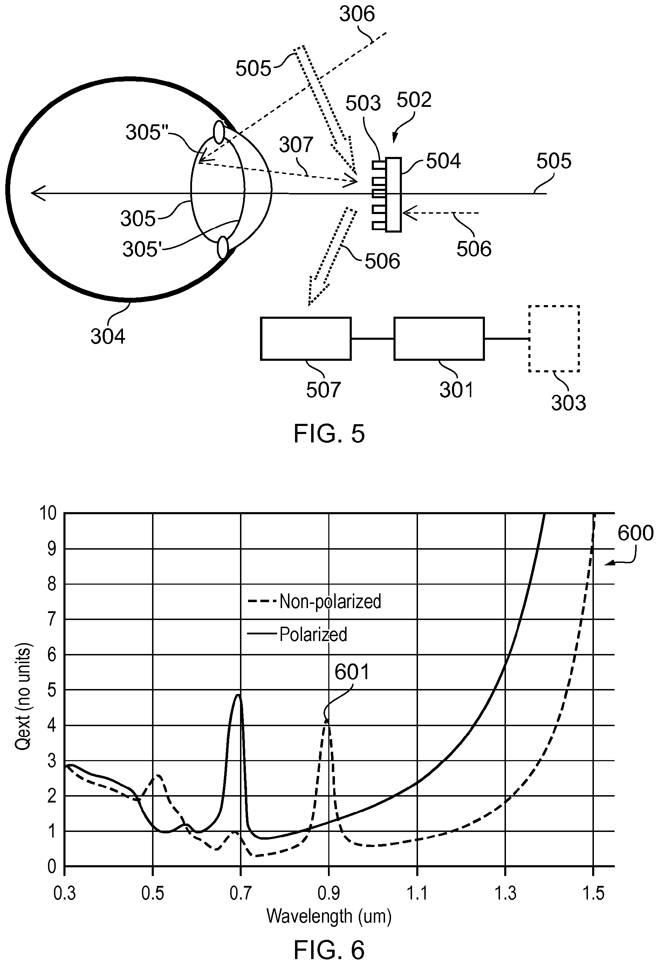

In block 201, the polarization of reflected light is measured/detected by performing at least one measurement of the polarization of the reflected light. Such detection and measurement may be performed by a polarization detector (an example of which is discussed in further detail below with respect to Figure 5). The measurements from the polarization detector may be provided to a controller (an example of which is discussed in further detail below with respect to Figures 3, 4 and 7).

In block 101 polarization measurements are received, e.g. at controller from the detector. In block 202 a determination of a change in the polarization of the reflected light is determined. Such a determination may take place in the controller. The determination of the change in polarization of the reflected light may relate to determining a change in a state of a characteristic of the polarization of the reflected light, such as one or more of:
determining a directional change of the polarization 202a, e.g. a change in the detected direction/orientation of the polarization of the reflected light;
determining a temporal change of the polarization 202b, e.g. a change in the detected polarization of the reflected light over time;
determining a spatial change of the polarization 202c, e.g. a change in the detected position/spatial location within a detector array of detected reflected light having a particular polarization state; and
determining a quantitative change of the polarization 202d, e.g. a change in an amount/flux of detected reflected light having a particular polarization state.

In block 203, the control of a focusing mechanism dependent on at least one polarization measurement (of block 102) comprises controlling the focussing element dependent on the determined change in polarization.

Figure 3 schematically illustrates a block diagram of an apparatus 300 according to an example of the present disclosure. The component blocks of Figure 3 are functional and the functions described may be performed by a single physical entity. Figure 3 focuses on the functional components necessary for describing the operation of the apparatus 300.

The apparatus comprises a controller 301 configured to receive measurements of the polarization of incident light 306 which is reflected 307 from a surface. Such polarization measurements maybe performed by a polarization detector 302, which detects and measures the polarization of light reflected from the surface.

The controller 301 is configured to control a focusing element 303 (shown in outline) wherein the control is dependent, at least in part, on the polarization measurements. The focusing element 303, may comprise part of the apparatus 300 itself (see also for example Figure 7A) or alternatively the focusing element may be a part of a device which is separate from the apparatus 300 (see for example with regards to Figure 7B). The control of the focusing element 303 may be via the generation of control signals which control the focussing mechanism, e.g. to cause the movement of one or more focussing element(s) or an adjustment of focussing element(s)'s refractive index or focal point.

Figure 3 shows the apparatus 300 detecting/measuring the polarization of light 306 reflected from a lens 305 of a user's eye 304. In the scenario shown in Figure 3, the user is looking at/focusing his/her gaze on a distant object A at a distance D₁ from the user's eye. Ambient light 306 incident to the user's lens 305 is reflected from a surface of the lens, such as the rear surface of the lens 305' and/or a front surface of the lens 305". The light 307 which is reflected from the at least one surface is detected by the detector 302.

One or more measurements P_{A} of the polarization of incident light 306 which is reflected 307 from the lens 305 may be taken. For example, at a particular time t=t₁, a plurality of polarization measurements P_{A1}, P_{A2} ...P_{An} of a plurality of reflected light beams 307_{A1}...307_{An} are measured, wherein the plurality of reflected light beams are from a plurality of incident light beams 306_{A1}...306_{An}.

One or more focussing mechanism control signals, CS_{A}, may be generated based on the measurements, i.e. one or more of a magnitude and direction of focus adjustment to be effected by the focussing mechanism may be based on the polarization measurements.

Figure 4 shows the apparatus 300 wherein the user is looking at an object B at a different distance D₂, which is located closer than the object A, i.e. D₂<<D₁. When a user's eye 304 seeks to focus on an object, the shape of the user's lens 305 changes. In the scenario of Figure 3, the user's eye is focused on a distant object A, as such the lens adopts an elongate/narrow/highly elliptical shape. Whereas, when focusing on a nearby object, as in Figure 4, the lens adopts a more circular/less elliptic shape.

Significantly, the inventor has appreciated that the shape of a user's lens changes when a user's eye adjusts its focus/point of focus, i.e. the shape of the user's lens depending on the distance of objects the user is focusing on. Moreover, the inventor has appreciated that the change of shape of the user's lens affects the polarization of light reflected from the user's lens. It is believed that the change in polarization may be due to an effective change in the angle of incidence and angle of reflectance at the surface of the lens as the shape of the lens changes, and thus the shape of the lens' surface changes.

In case A of Figure 3, when the user is focused on an object A located at distance D1, the lens would have a first particular shape. This would give rise to a first detected polarization profile/characteristic/signature for a first set of incident beams of light which are reflected from the lens' surface having an angle of incidence corresponding to the Brewster angle and are thus have reflected rays with which are polarized.

However, in case B of Figure 4, when the user changes his/her focus to an object B located at a different distance D2, the lens' shape changes to a second different particular shape. This would give rise to a second detected polarization profile/characteristic/signature for a second set of incident beams of light which are reflected from the lens' surface having an angle of incidence corresponding to the Brewster angle and are thus have reflected rays with which are polarized (it being noted that the lens' surface in case B would have a differing shape/orientation to that of case A and thus the detected polarization profile/characteristic/signature for case B would differ from that of case A).

For example, particular incident light rays in Figure 3 which happen to have an angle of incidence equal to the Brewster angle giving rise to reflected polarized rays that detected by the detector may no longer be polarized in the scenario of Figure 4 where the lens shape has changed. Likewise, particular incident light rays which previously were not polarized in the scenario of Figure 3 but, following a change in shape of the lens in Figure 4, may now be reflected from a surface of the differing shaped lens with an angle of incidence equal to the Brewster angle such that the reflected rays are now polarized.

Certain examples of the present disclosure make use of an new appreciation of a relationship between the polarization characteristics of light reflected from a part of the user's eye (e.g. lens 305 or ciliary muscle 308) and the shape of the part of the user's eye, as well as the fact that the shape of the eye part is itself dependent on the user's focus/ point of focus. Thus, the measured/detected polarization profile of reflected light may be indicative of a user's focus/ point of focus.

Moreover, the inventor has appreciated that a change in user's focus gives rise to a change in the shape of the eye part, which itself gives rise to a change in the polarization characteristics of light reflected from the eye part. Thus, the inventor has appreciated that a change in the polarization characteristics may be indicative of a change in the user's focus. Furthermore, where the change in polarization is zero, thus is indicative of there being no change in the shape of the eye part and thus one can deduce that a user's focus is stable/fixed on a particular object at a fixed distance/focal length.

Accordingly, certain examples of present invention take advantage of the human's eye's natural ability to focus on objects, which is a function predominantly performed by the lens of the eye based on the shape of the lens. This, in combination with the insight that such a change in shape of the lens gives rise to a change in detected polarization characteristics of light reflected from the lens, is used to control a focusing mechanism, wherein the control is dependent on the polarization, such as a change in the detected polarization measurements.

In Figure 4, the apparatus 300 measures/detects one or more measurements P_{B} of the polarization of incident light 306_{B} which is reflected 307_{B} from the lens 305. For example, at a particular time t=t₂, a plurality of polarization measurements P_{B1}, P_{B2} ...P_{Bn} of a plurality of reflected light beams 307_{B1}...307_{Bn} are measured, wherein the plurality of reflected light beams are from a plurality of incident light beams 306_{B1}...306_{Bn}. One or more focussing mechanism control signals, CS_{B}, may be generated based on the polarization measurements, i.e. one or more of a magnitude and direction of focus adjustment to be effected by the focussing mechanism may be based on the polarization measurements.

The control signal CS₂ may be generated which is dependent on a determined change in the polarization characteristic, e.g. a change in the detected polarization characteristics between the scenario of Figure 3 and the scenario of Figure 4, namely ΔP = P_{A} - P_{B} , or ΔP = {P_{A1}, P_{A2} ...P_{An}} - {P_{B1}, P_{B2} ...P_{Bn}}.

In certain examples, the detection of a change in the polarization of light reflected from the user's lens comprises detecting/measuring at a first time t=t₁, a first set P_{A} of one or more polarization characteristics {P_{A1}, P_{A2} ...P_{An}} and detecting, at a second time t=t₂, a second set P_{B} of one or more polarization characteristics {P_{B1}, P_{B2} ...P_{Bn}}. In this manner, a temporal variation of the polarization characteristics may be determined based on a difference between the first and second sets of polarization characteristics.

In certain examples, the detection/measurement of polarization states is carried out with a detector having a two-dimensional detector array such that the detection of polarization comprise: quantitative measurements of the amount of reflected light detected having a particular polarization state/direction as well as enabling a characterisation of the polarization based on its detected position within the detector array. I.e. the polarization measurements may include an indication of a position/location within the detector of the detection of the various polarizations states, such as x, y coordinates within the detector corresponding to the location of a particular measured/detected polarization state. In this manner, a spatial characteristic and spatial variation of the polarization of reflective light may be determined based on the detected positions within the detector array of the various polarization states.

Figure 5 shows an example of a detector 502 which may be used with apparatuses of the present disclosure. The detector 502 comprises one or more nanoparticles 503 that are provided on a substrate 504. The nanoparticles are configured such that an optical property of the nanoparticle is sensitive to/responsive to a polarization state of incident light 307. For example, the nanoparticles may be configured (not least for example based on their chemical composition as well as structural arrangement and alignment) such that an optical property, e.g. absorption and scattering characteristics, of the nanoparticles is affected by/dependent on the polarization state of light 307 incident to the nanoparticles 503.

Measurements of the values of the optical property of the array of nanoparticles can be detected by a further detector 507. For example, the further detector 507 may correspond to a photo electric converter, a photo-sensitive detector or a CCD device configured to measure and detect changes in the absorption and scattering characteristics of the array of nanoparticles 503 by detecting ambient light 505 which is reflected off the array of nanoparticles 503, such reflected light 506 being detected and measured by the further detector 507.

Measurements of the values of the optical property of the nanoparticles 501 may comprise a quantitative measurement of the optical property values themselves as well as a spatial measurement associated with each measurement value, i.e. a position/location within the further detector 507 of the measurement of the various optical property values of the detector 502 and its array of nanoparticles 503. In this manner, a spatial variation of the polarization of reflected light 307 may be determined based on a spatial variation of the measurements positions of reflected light 506 within the further detector array 507 of the various optical property measurements. Likewise, measurements of the optical property of the detector array 502 may be done at two or more differing times so to enable a temporal variation of the polarization measurements to be determined.

The detector 502 and its arrangement of nanoparticles 503 may be configured so as to have plasmonic resonances in the infrared region (around 700 nanometres). This prevents visible and ultra-violet light from exciting the nanoparticle array. Also, the nano particular array may be configured such that only incident light 307 which is substantially perpendicular to the longitudinal axis of the nanoparticle array 503 can be used to excite the plasmonic resonances. The material of the substrate 504 may be selected so as to prevent certain wavelengths of light 506 passing through the substrate and interacting with the nanoparticle array 503 (e.g. a glass substrate would prevent the passage of infrared light there through due to the glass substrate absorbing infrared radiation), whereas other wavelengths of light such as visible light 505 are able to pass through the detector 502 and its substrate 502 and nanoparticle array 501 for viewing by the user's eye 304, i.e. the detector may be configured to be transparent to certain wavelengths of light, not least light in the visible part of the spectrum. Such measures help ensure that only polarization variations from infrared light reflected from the lens excites the plasmonic resonances and that the optical signature detected by the detector 507 of the optical characteristics of the nanoparticle array 503 are indicative of polarization characteristics just of light 307 reflected from the user's lens 501 (such polarization characteristics being indicative of the shape of the user's lenses which itself is indicative of a focus point of the user's eye/focal viewing point).

The geometry of the array and the alignment of nano-rods may be configured so as to be optimised for operation in the infrared region, e.g. at around 700 nanometres, and also be optimised for the detection of polarization variations for light which is incident at 90° (i.e. orthogonal) to the array and plane of the substrate. This can help ensure that only polarization variations from light 307 reflected from the lens is detected, as opposed to other incident light e.g. ambient light/irradiation 505 incident at an obtuse angle. Such a configuration of the nanoparticles may comprise an appropriate selection of the material of the nanoparticles, for example gold or zinc oxide (as well as the structure and arrangement of the nanoparticles, for example providing the nanoparticles as nano-rods which are aligned perpendicular to a direction of incident reflected light.

Various examples make use of a plasmonic phenomenon of a nanoparticle array whose extreme sensitivity to polarization variations enables the use of ambient light 306 and ambient infrared light thereby negating requirement for additional hardware such as external dedicated non-natural sources of illumination and an optical system (e.g. mirrors and lenses) for the same. This enables devices according to the present disclosure to be versatile, non-cumbersome and usable in head mountable/wearable/portable devices. In some examples the requirement for complex image processing techniques and analysis may be reduced/negated since only a variation in polarization characteristics need be detected.

Examples of the present disclosure may be used for a variety of applications and devices, not least glasses, goggles, binoculars and eyeglass lenses (when using adaptive refractive index materials) as well as in the healthcare profession to provide ongoing spectral bio-imaging and monitoring of the eye. Examples may be used for both near and far field applications/focussing. Examples may designed to operate for multiple users on multiple devices and may provide a real-time autofocussing detector.

Figure 6 illustrates a graph 600 showing modelled results indicating the responsiveness/sensitivity of the excitation of plasmonic resonances to the polarization of incident light. An extinction spectrum (relating to the amount of light absorbed and scattered) of a gold nano-rod having a 30 nanometre diameter and 280 nanometre length is shown. The extinction spectrum was modelled in water with incident light polarized along a longitudinal axis of the nano-rod (shown with the solid line) and secondly with non-polarized light (shown with the dashed line). It can be seen that an additional high order mode 601 is excited in the non-polarized state because previously forbidden resonances (for the polarized case) are now able to be excited. In this proof of concept modelling, a gold nanoparticle was considered. It is to be appreciated that nanoparticles of other compositions could be considered not least for example zinc oxide (ZnO) nano-rods which are transparent in the visible spectrum and have plasmonic resonances operating in the infrared range.

The use of an array of nanoparticles providing plasmonic resonance excitation may provide an extremely sensitive polarization detector. Advantageously, this enables the incident light source that is used for the detector 302 to be merely ambient light rather than requiring a dedicated specific illumination source. Thus, examples may make use of ambient light, and in particular ambient infrared light, as the 'probing' incident light source, reflections of which from a user's lens are detected by the detector 502.

Examples of the present disclosure may take the form of a method, an apparatus or a computer program. Accordingly, examples may be implemented in hardware, software or a combination of hardware and software.

Examples of the present disclosure are described using flowchart illustrations and schematic block diagrams. It will be understood that each block (of the flowchart illustrations and block diagrams), and combinations of blocks, can be implemented by appropriately configured means, devices, mechanisms or hardware as well as software, e.g. computer program instructions of a computer program. These program instructions may be provided to one or more processor(s), processing circuitry or controller(s) such that the instructions which execute on the same create means for causing/implementing the functions specified in the block or blocks. The computer program instructions may be executed by the processor(s) to cause a series of operational steps/actions to be performed by the processor(s) to produce a computer implemented process such that the instructions which execute on the processor(s) provide steps for implementing the functions specified in the block or blocks.

Accordingly, the blocks support: combinations of means for performing the specified functions; combinations of actions for performing the specified functions; and computer program instructions/algorithm for performing the specified functions. It will also be understood that each block, and combinations of blocks, can be implemented by special purpose hardware-based systems which perform the specified functions or actions, or combinations of special purpose hardware and computer program instructions.

Figure 7 schematically illustrates an apparatus 700 comprising various means configured to cause the apparatus to perform the above described methods.

The apparatus 700 comprises a controller 301. The controller 301 is configured to receive polarization measurements P_{A}, P_{B} from a detector 302 (which may form a part of the apparatus or may be part or another separate device) and is configured to control a focussing mechanism 303 (which may form a part of the apparatus or may be part or another separate device), wherein the control of the focussing element is dependent on the polarization measurements.

Implementation of the controller 301 can be in hardware alone (e.g. processing circuitry comprising one or more processors and memory circuitry comprising one or more memory elements), have certain aspects in software including firmware alone or can be a combination of hardware and software (including firmware).

The controller 301 may be implemented using instructions that enable hardware functionality, for example, by using executable computer program instructions in a general-purpose or special-purpose processor that may be stored on a computer readable storage medium (disk, memory etc.) or carried by a signal carrier to be performed by such a processor.

In the illustrated example, the apparatus 700 comprises a controller 301 which is provided by a processor 702 and memory 703. Although a single processor and a single memory are illustrated in other implementations there may be multiple processors and/or there may be multiple memories some or all of which may be integrated/removable and/or may provide permanent/semi-permanent/ dynamic/cached storage.

The memory 703 stores a computer program 704 comprising computer program instructions 705 that control the operation of the apparatus when loaded into the processor 702. The computer program instructions provide the logic and routines that enable the apparatus to perform the methods presently described.

The at least one memory 703 and the computer program instructions 705 are configured to, with the at least one processor 702, cause the apparatus 700 at least to perform the method described, not least for example with respect to Figures 1 and 2.

The processor 702 is configured to read from and write to the memory 703. The processor 702 may also comprise an input interface 706 via which data, such as polarization measurements, and/or commands are input to the processor 702, and an output interface 707 via which data, such as control signals for the focussing mechanism are output by the processor 702.

The computer program may arrive at the apparatus 700 via any suitable delivery mechanism 711. The delivery mechanism 711 may be, for example, a non-transitory computer-readable storage medium, a computer program product, a memory device, a record medium such as a compact disc read-only memory, or digital versatile disc, or an article of manufacture that tangibly embodies the computer program 704. The delivery mechanism may be a signal configured to reliably transfer the computer program 704.

The apparatus 700 may receive, propagate or transmit the computer program 704 as a computer data signal.

The apparatus may be a part of a device or system 710 which additionally includes the detector 302 and the focussing mechanism 303.

The apparatus 700 and/or device or system 710 may, for example, be one or more of: a head mountable device, a wearable device, a portable device, a handheld device, a mobile cellular telephone, a wireless communications device, or an optical device. The apparatus 700 may be a module or chipset for use in any of the foregoing.

Although examples of the apparatus have been described above in terms of comprising various components, it should be understood that the components may be embodied as or otherwise controlled by a corresponding processing element or processor of the apparatus. In this regard, each of the components described above may be one or more of any device, means or circuitry embodied in hardware, software or a combination of hardware and software that is configured to perform the corresponding functions of the respective components as described in greater detail below.

References to 'computer-readable storage medium', 'computer program product', 'tangibly embodied computer program' etc. or a 'controller', 'computer', 'processor' etc. should be understood to encompass not only computers having different architectures such as single /multi- processor architectures and sequential (Von Neumann)/parallel architectures but also specialized circuits such as field-programmable gate arrays (FPGA), application specific circuits (ASIC), signal processing devices and other devices. References to computer program, instructions, code etc. should be understood to encompass software for a programmable processor or firmware such as, for example, the programmable content of a hardware device whether instructions for a processor, or configuration settings for a fixed-function device, gate array or programmable logic device etc.

As used in this application, the term 'circuitry' refers to all of the following:
(a) hardware-only circuit implementations (such as implementations in only analogue and/or digital circuitry) and
(b) to combinations of circuits and software (and/or firmware), such as (as applicable): (i) to a combination of processor(s) or (ii) to portions of processor(s)/software (including digital signal processor(s)), software, and memory(ies) that work together to cause an apparatus, such as a mobile phone or server, to perform various functions) and
(c) to circuits, such as a microprocessor(s) or a portion of a microprocessor(s), that require software or firmware for operation, even if the software or firmware is not physically present.

This definition of 'circuitry' applies to all uses of this term in this application, including in any claims. As a further example, as used in this application, the term "circuitry" would also cover an implementation of merely a processor (or multiple processors) or portion of a processor and its (or their) accompanying software and/or firmware. The term "circuitry" would also cover, for example and if applicable to the particular claim element, a baseband integrated circuit or applications processor integrated circuit for a mobile phone or a similar integrated circuit in a server, a cellular network device, or other network device."

In one example, the apparatus 700 is embodied on a computing device that may be: portable, head mountable, wearable or handheld. The computing device may additionally provide one or more audio/text/video communication functions (e.g. telecommunication, video-communication, and/or text transmission (Short Message Service (SMS)/ Multimedia Message Service (MMS)/emailing) functions), interactive/non-interactive viewing functions (e.g. web-browsing, navigation, TV/program viewing functions), music recording/playing functions (e.g. Moving Picture Experts Group-1 Audio Layer 3 (MP3) or other format and/or (frequency modulation/amplitude modulation) radio broadcast recording/playing), downloading/sending of data functions, image capture function (e.g. using a (e.g. in-built) digital camera), and gaming functions.

Examples of the present disclosure provide both a method and corresponding apparatus consisting of various modules, means or circuitry that provide the functionality for performing the actions of the method. The modules, means or circuitry may be implemented as hardware, or may be implemented as software or firmware to be performed by a computer processor. In the case of firmware or software, examples of the present disclosure can be provided as a computer program product including a computer readable storage structure embodying computer program instructions (i.e. the software or firmware) thereon for performing by the computer processor.

The apparatus may be provided in a module. As used here 'module' refers to a unit or apparatus that excludes certain parts/components that would be added by an end manufacturer or a user.

The apparatus may be provided in an electronic device, for example, a head mountable device or portable hand held device, according to exemplary embodiments of the present disclosure. It should be understood, however, that these are merely illustrative of an electronic device that would benefit from examples of implementations of the present disclosure and, therefore, should not be taken to limit the scope of the present disclosure to the same. While in certain implementation examples the apparatus may be provided in a head mountable device or portable hand held device, other types of electronic devices, such as, but not limited to, wearable computing devices, portable digital assistants (PDAs), mobile computers, desktop computers, televisions, gaming devices, laptop computers, tablet devices, cameras, video recorders, imaging devices, GPS devices and other types of electronic systems, may readily employ examples of the present disclosure. Furthermore, devices may readily employ examples of the present disclosure regardless of their intent to provide mobility.

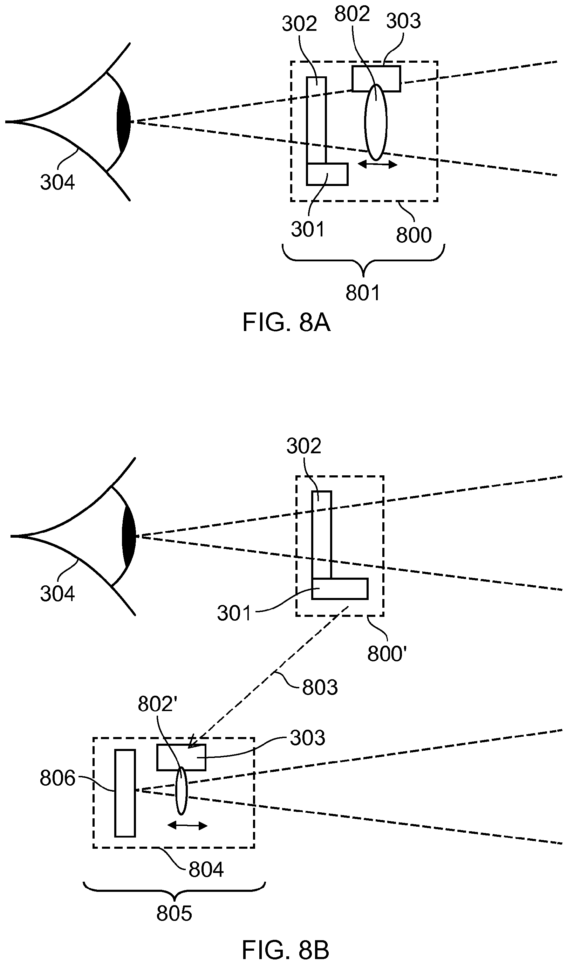

Figure 8A schematically illustrates a further example of an apparatus 800 according to an example of the present disclosure. The apparatus 800 comprises each of the controller 301, the detector 302 as well as the focussing mechanism 303 for adjusting the focussing of a focus element 802 of an optical system 801 whose focus is to be controlled. The apparatus 700 may be configured so as to be situated, in use, in front of a user's eye, i.e. the apparatus may be configured as an optical system through which a user may view therethrough so that the apparatus may provide an auto-focus adjusting mechanism for adjusting the focus of the optical system 802 which the user is looking through. In some examples, where a plurality of nanoparticles are used for the detector 302, the detector may be transparent to visible light and provided as an optical component of the optical system 801, i.e. the nanoparticles may be deposited on an optical component of the optical system, e.g. a glass lens.

The apparatus 800 of Figure 8A may correspond to a head-mountable device such as glasses or goggles or may be a hand portable device not least for example monoculars, binoculars or an image capturing device. The device 800 need not necessarily be hand portable, for example, the device could correspond to a telescope.

Figure 8B shows an alternative apparatus 800' which comprises just the controller 301 and the detector 302. The focusing element 303 which receives control signals (indicated by arrow 803) from the controller 301 is provided as a separate device 804. The separate device 804 may correspond to an optical system 805 whose focus may be controlled by the apparatus 800'. The optical system 805 may be an image capturing device having an image sensor 806 and one or more lenses 802', where the movement of the one or more lenses 802'can be driven by a motor/actuator of the focussing mechanism based on the control of the apparatus 800'.

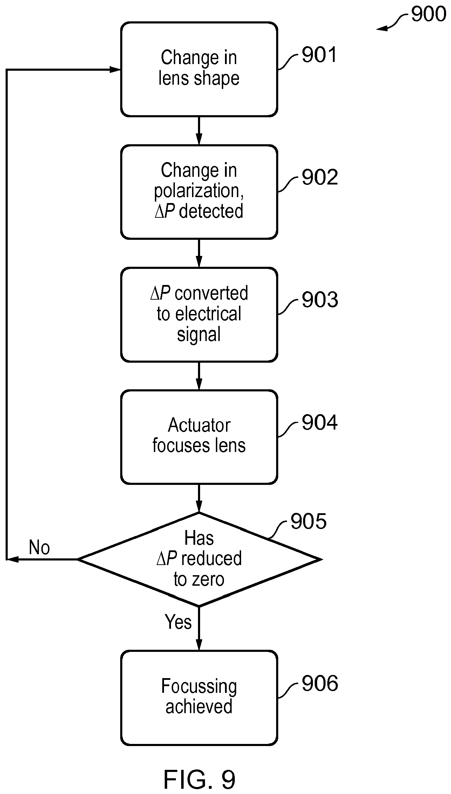

Figure 9 schematically illustrates a flowchart 900 depicting operation of a real time autofocusing detector.

In block 902, following a change in a user's lens shape in block 901, a change in polarization, ΔP, of light reflected from the lens is detected. In block 903, the detected change in polarization ΔP is converted to an electrical signal for controlling a focussing mechanism, such as an actuator for a lens. In block 904, the actuator focuses the lens by moving the lens in dependence on the received electrical signal (which itself is dependent on the detected change in polarization). In block 905, a determination is made as to whether or not a change in polarization has been reduced to 0.

A detected change in polarization being zero is indicative of there being no change in the shape of the lens. No change in shape of the lens is indicative of the lens not re-focussing or the eye attempting to focus. Thus, when the detected change in polarization is zero, one can deduce the user's focus is stable/fixed on a particular object at a fixed distance/focal length and that the eye is in correct focus / the user is not perceiving a blurry image.

If the change in polarization has been reduced to 0 the focusing of the lens has been achieved and the operation ends at block 906. If the change in polarization has not been reduced to 0 then the operation reverts back to repeat steps 901 to 906.

Essentially, if the user's eye is attempting to focus on an object, the lens geometry will vary, which alters the polarization of light reflected from the lens, i.e. gives rise to a change in polarization ΔP. The ΔP detected is used to create a control signal to drive the focussing mechanism. For example, where the polarization detector is a nanoparticle array as described above which has an optical signature (i.e. related to its absorption and scatting characteristics) which is dependent on the polarization of incident light, a photoelectric converter may convert the optical signature to a control signal for driving the focusing mechanism. The driving of the focus mechanism would stop when the eye is in focus (and in a stable/fixed shape) and thus ΔP is reduced to zero.

The blocks illustrated in Figures 1, 2 and 9 may represent actions in a method and/or sections of instructions/code in the computer program.

It will be understood that each block and combinations of blocks, can be implemented by various means (e.g. the example apparatuses described above) such as hardware, firmware, and/or software including one or more computer program instructions. For example, one or more of the procedures described above may be embodied by computer program instructions. In this regard, the computer program instructions which embody the procedures described above may be stored by a memory storage device and performed by a processor.

As will be appreciated, any such computer program instructions may be loaded onto a computer or other programmable apparatus (i.e., hardware) to produce a machine, such that the instructions when performed on the programmable apparatus create means for implementing the functions specified in the blocks. These computer program instructions may also be stored in a computer-readable medium that can direct a programmable apparatus to function in a particular manner, such that the instructions stored in the computer-readable memory produce an article of manufacture including instruction means which implement the function specified in the blocks. The computer program instructions may also be loaded onto a programmable apparatus to cause a series of operational actions to be performed on the programmable apparatus to produce a computer-implemented process such that the instructions which are performed on the programmable apparatus provide actions for implementing the functions specified in the blocks.

The illustration of a particular order to the blocks does not necessarily imply that there is a required or preferred order for the blocks and the order. Furthermore, it may be possible for some blocks to be omitted. Although functions have been described with reference to certain features, those functions may be performable by other features.

Although features have been described with reference to certain examples, those features may also be present in other examples.

Although various examples of the present disclosure have been described in the preceding paragraphs with reference to various examples, it should be appreciated that modifications to the examples given can be made without departing from the scope of the invention as set out in the claims.

The term 'comprise' is used in this document with an inclusive not an exclusive meaning. That is any reference to X comprising Y indicates that X may comprise only one Y or may comprise more than one Y. If it is intended to use 'comprise' with an exclusive meaning then it will be made clear in the context by referring to "comprising only one ..." or by using "consisting".

In this description, the wording 'connect', 'couple' and 'communication' and their derivatives mean operationally connected/coupled/in communication. It should be appreciated that any number or combination of intervening components can exist (including no intervening components).

In this description, reference has been made to various examples. The description of features or functions in relation to an example indicates that those features or functions are present in that example. The use of the term 'example' or 'for example' or 'may' in the text denotes that such features or functions are present in at least the described example and that they can be, but are not necessarily, present in some or all other examples. Thus 'example', 'for example' or 'may' refers to a particular instance in a class of examples. A property of the instance can be a property of only that instance or a property of the class or a property of a sub-class of the class.

In this description, references to "a/an/the" [feature, element, component, means ...] are to be interpreted as "at least one" [feature, element, component, means ...] unless explicitly stated otherwise.

In the above description, the apparatus described may comprise an apparatus which in some other embodiments comprises a distributed system of apparatus, for example, wherein one or more of the detector and/or focusing element to be controlled are separate of the apparatus. In examples of embodiments where an apparatus provided forms (or a method is implemented as) a distributed system, each apparatus forming a component and/or part of the system provides (or implements) one or more features which collectively implement an example of the present disclosure. In some examples of embodiments, an apparatus is re-configured by an entity other than its initial manufacturer to implement an example of the present disclosure by being provided with additional software, for example by a user downloading such software, which when executed causes the apparatus to implement an example of the present disclosure (such implementation being either entirely by the apparatus or as part of a system of apparatus as mentioned hereinabove).

## Claims

1. A method comprising : receiving at least one measurement of a polarization (P_{A}) of light (307) reflected from at least one surface (305', 305") of a part of a user's eye (304);
determining a change in the polarization (ΔP) of the reflected light with respect to the polarization of the incident light (306); and
controlling a focusing mechanism (303) dependent, at least in part, on the determined change in polarization.

2. The method of claim 1, wherein the at least one surface comprises a lens (305) of a user's eye.

3. The method of claim 1, wherein the determined change in polarization comprises one or more of:
a change in direction of polarization;
a temporal change of polarization; and
a spatial change of polarization.

4. The method of any one or more of the previous claims comprising using a detector (302) configured to one or more of:
measure the polarization of light reflected from the at least one surface;
detect the polarization of light reflected from the at least one surface; and
detect a change in the polarization of reflected light.

5. The method of claim 4, wherein the detector is configured such that an optical property of the detector is adjustable in response to a polarization state of light incident to the detector, and wherein the method comprises:
measuring the optical property of the detector for determining information related to the polarization of light incident on the detector.

6. The method of any one or more of the previous claims, wherein the reflected light comprises one or more of:
reflected ambient light; and
reflected infrared light.

7. An apparatus (300) comprising means (301) configured to cause the apparatus at least to perform:
receiving at least one measurement of a polarization (P_{A}) of light (307) reflected from at least one surface (305', 305") of a part of a user's eye (304);
determining a change in the polarization (ΔP) of the reflected light with respect to the polarization of the incident light (306); and
controlling a focusing mechanism (303) dependent, at least in part, on the determined change in polarization.

8. The apparatus of claim 7 further comprising means configured to cause the apparatus at least to perform:
the method as claimed in any one or more of claims 2- 6.

9. The apparatus of claim 7 or 8, further comprising a detector (302) for measuring the polarization of light reflected from the surface.

10. The apparatus of claim 9, wherein the detector comprises a plurality of nanoparticles (503) configured to have plasmonic resonances that are excitable in response to one or more of:
a polarization state of incident light; and
incident infrared light.

11. The apparatus of claim 10, further comprising a detector (507) configured to measure the plasmonic resonance excitations.

12. The apparatus of any one or more of previous claims 10 to 11, wherein the plurality of nanoparticles comprise nano-rods configured so as to be aligned substantially perpendicularly to a direction of the reflected light.

13. The apparatus of any one or more of previous claims 7 to 12 further comprising an optical system (801) for user viewing therethrough, wherein the focusing mechanism is configured to adjust a focus of the optical system.

14. A device (800) comprising:
the apparatus of any one or more of previous claims 7 to 13, and wherein the device is one or more of:
a head mountable device, a wearable device, a portable device, a handheld device, an optical device and a wireless communications device.

15. Computer program instructions (705) for causing an apparatus (300) as claimed in any one of claims 7 - 13 to perform the method as claimed in any one or more of claims 1 - 6.

## Patentansprüche

1. Verfahren umfassend:
Empfangen zumindest einer Messung einer Polarisation (P_{A}) von Licht (307), das von mindestens einer Oberfläche (305', 305") eines Teils des Auges eines Benutzers (304) reflektiert wird;
Bestimmen einer Änderung der Polarisation (ΔP) des reflektierten Lichts in Bezug auf die Polarisation des einfallenden Lichts (306); und
Steuern eines Fokussiermechanismus (303), der zumindest teilweise von der bestimmten Änderung der Polarisation abhängig ist.

2. Verfahren nach Anspruch 1, wobei die mindestens eine Oberfläche eine Linse (305) des Auges eines Benutzers umfasst.

3. Verfahren nach Anspruch 1, wobei die bestimmte Änderung der Polarisation eine oder mehrere der folgenden umfasst:
eine Änderung der Polarisationsrichtung;
eine zeitliche Änderung der Polarisation; und
eine räumliche Veränderung der Polarisation.

4. Verfahren nach einem oder mehreren der vorstehenden Ansprüche, umfassend die Verwendung eines Detektors (302), der für eine oder mehrere der folgenden Aktionen konfiguriert ist:
Messen der Polarisation von Licht, das von der mindestens einen Oberfläche reflektiert wird;
Erkennen der Polarisation von Licht, das von der mindestens einen Oberfläche reflektiert wird; und
Erkennen einer Änderung der Polarisation des reflektierten Lichts.

5. Verfahren nach Anspruch 4, wobei der Detektor so konfiguriert ist, dass eine optische Eigenschaft des Detektors als Reaktion auf einen Polarisationszustand von Licht, das auf den Detektor einfällt, einstellbar ist, und wobei das Verfahren umfasst:
Messen der optischen Eigenschaft des Detektors für die Bestimmung von Informationen in Bezug auf die Polarisation von Licht, das auf den Detektor einfällt.

6. Verfahren nach einem oder mehreren der vorstehenden Ansprüche, wobei das reflektierte Licht eines oder mehrere umfasst von:
reflektiertes Umgebungslicht; und
reflektiertes Infrarotlicht.

7. Vorrichtung (300) umfassend Mittel (301), die dafür konfiguriert sind, die Vorrichtung zu veranlassen, mindestens auszuführen:
Empfangen zumindest einer Messung einer Polarisation (P_{A}) von Licht (307), das von mindestens einer Oberfläche (305', 305") eines Teils des Auges eines Benutzers (304) reflektiert wird;
Bestimmen einer Änderung der Polarisation (ΔP) des reflektierten Lichts in Bezug auf die Polarisation des einfallenden Lichts (306); und
Steuern eines Fokussiermechanismus (303), der zumindest teilweise von der bestimmten Änderung der Polarisation abhängig ist.

8. Vorrichtung nach Anspruch 7, die ferner Mittel umfasst, die dafür konfiguriert sind, die Vorrichtung zu veranlassen, mindestens auszuführen:
das Verfahren nach einem oder mehreren der Ansprüche 2 bis 6.

9. Vorrichtung nach Anspruch 7 oder 8, die ferner einen Detektor (302) zum Messen der Polarisation von Licht umfasst, das von der Oberfläche reflektiert wird.

10. Vorrichtung nach Anspruch 9, wobei der Detektor eine Vielzahl von Nanopartikeln (503) umfasst, die so konfiguriert sind, dass sie plasmonische Resonanzen aufweisen, die als Reaktion auf eines oder mehrere der folgenden anregbar sind:
ein Polarisationszustand des einfallenden Lichts; und
einfallendes Infrarotlicht.

11. Vorrichtung nach Anspruch 10, die ferner einen Detektor (507) umfasst, der dafür konfiguriert ist, die plasmonischen Resonanzanregungen zu messen.

12. Vorrichtung nach einem oder mehreren der vorstehenden Ansprüche 10 bis 11, wobei die Vielzahl von Nanopartikeln Nanostäbchen umfasst, die so konfiguriert sind, dass sie im Wesentlichen senkrecht zu einer Richtung des reflektierten Lichts ausgerichtet werden.

13. Vorrichtung nach einem oder mehreren der vorstehenden Ansprüche 7 bis 12, die ferner ein optisches System (801) für die Betrachtung durch den Benutzer umfasst, wobei der Fokussiermechanismus dafür konfiguriert ist, einen Brennpunkt des optischen Systems einzustellen.

14. Gerät (800) umfassend:
die Vorrichtung nach einem oder mehreren der vorstehenden Ansprüche 7 bis 13, und wobei das Gerät eines oder mehrere ist von:
ein am Kopf zu befestigendes Gerät, ein am Körper zu tragendes Gerät, ein tragbares Gerät, ein Handgerät, ein optisches Gerät und ein drahtloses Kommunikationsgerät.

15. Computerprogrammbefehle (705) zum Veranlassen einer Vorrichtung (300) nach einem der Ansprüche 7 bis 13, das Verfahren nach einem oder mehreren der Ansprüche 1 bis 6 durchzuführen.

## Revendications

1. Procédé qui comprend :
la réception d'au moins une mesure d'une polarisation (P_{A}) d'une lumière (307) réfléchie par au moins une surface (305', 305'') d'une partie d'un œil d'un utilisateur (304) ;
la détermination d'un changement de polarisation (ΔP) de la lumière réfléchie par rapport à la polarisation de la lumière incidente (306) ; et
le contrôle d'un mécanisme de mise au point (303) qui dépend, au moins en partie, du changement de polarisation déterminé.

2. Procédé selon la revendication 1, dans lequel la au moins une surface comprend un cristallin (305) d'un œil d'un utilisateur.

3. Procédé selon la revendication 1, dans lequel le changement déterminé de polarisation comprend un ou plusieurs parmi :
un changement de direction de polarisation ;
un changement temporel de polarisation ; et
un changement spatial de polarisation.

4. Procédé selon l'une ou plusieurs des revendications précédentes, qui comprend l'utilisation d'un détecteur (302) configuré pour effectuer une ou plusieurs parmi :
la mesure de la polarisation de la lumière réfléchie par la au moins une surface ;
la détection de la polarisation de la lumière réfléchie par la au moins une surface ; et
la détection d'un changement de polarisation de la lumière réfléchie.

5. Procédé selon la revendication 4, dans lequel le détecteur est configuré de sorte qu'une propriété optique du détecteur soit ajustable en réponse à un état de polarisation de la lumière incidente sur le détecteur, et dans lequel le procédé comprend :
la mesure de la propriété optique du détecteur afin de déterminer des informations relatives à la polarisation de la lumière incidente sur le détecteur.

6. Procédé selon l'une ou plusieurs des revendications précédentes, dans lequel la lumière réfléchie comprend une ou plusieurs parmi :
une lumière ambiante réfléchie ; et
une lumière infrarouge réfléchie.

7. Appareil (300) qui comprend un moyen (301) configuré pour amener l'appareil à effectuer au moins :
la réception d'au moins une mesure d'une polarisation (P_{A}) d'une lumière (307) réfléchie par au moins une surface (305', 305") d'une partie d'un œil d'un utilisateur (304) ;
la détermination d'un changement de polarisation (ΔP) de la lumière réfléchie par rapport à la polarisation de la lumière incidente (306) ; et
le contrôle d'un mécanisme de mise au point (303) qui dépend, au moins en partie, du changement de polarisation déterminé.

8. Appareil selon la revendication 7, qui comprend en outre un moyen configuré pour amener l'appareil à exécuter au moins :
le procédé selon l'une ou plusieurs des revendications 2 à 6.

9. Appareil selon la revendication 7 ou 8, qui comprend en outre un détecteur (302) destiné à mesurer la polarisation de la lumière réfléchie par la surface.

10. Appareil selon la revendication 9, dans lequel le détecteur comprend une pluralité de nanoparticules (503) configurées pour présenter des résonances plasmoniques qui sont excitables en réponse à un ou plusieurs parmi :
un état de polarisation de la lumière incidente ; et
une lumière infrarouge incidente.

11. Appareil selon la revendication 10, qui comprend en outre un détecteur (507) configuré pour mesurer les excitations de résonances plasmoniques.

12. Appareil selon l'une ou plusieurs des revendications précédentes 10 à 11,
dans lequel la pluralité de nanoparticules comprend des nano-tiges configurées de façon à être alignées de manière sensiblement perpendiculaire à une direction de la lumière réfléchie.

13. Appareil selon l'une ou plusieurs des revendications précédentes 7 à 12, qui comprend en outre un système optique (801) destiné à permettre à un utilisateur de regarder à travers, dans lequel le mécanisme de mise au point est configuré pour régler une mise au point du système optique.

14. Dispositif (800) qui comprend :
l'appareil selon l'une ou plusieurs des revendications précédentes 7 à 13, et dans lequel le dispositif est un ou plusieurs parmi :
un dispositif qui peut être monté sur une tête, un dispositif portatif, un dispositif portable, un dispositif à porter, un dispositif optique et un dispositif de communication sans fil.

15. Instructions de programme informatique (705) pour amener un appareil (300) selon l'une quelconque des revendications 7 à 13 à exécuter le procédé selon l'une ou plusieurs des revendications 1 à 6.
